# EUROPEAN PATENT SPECIFICATION

(11) **EP 2 566 528 B1**
(45) Date of publication and mention of the grant of the patent: **30.11.2016**
(21) Application number: 11720063.4
(22) Date of filing: 02.05.2011
(51) Int. Cl.: A61L 27/56

(54) **MEDICAL IMPLANT**
MEDIZINISCHES IMPLANTAT
IMPLANT MÉDICAL

(30) Priority: 05.05.2010 DE 102010020662
(43) Date of publication of application: 13.03.2013
(73) Proprietor: AESCULAP AG, 78532 Tuttlingen (DE)
(72) Inventor: BLENDER, Bernd, 78532 Tuttlingen (DE); ODERMATT, Erich, CH-8200 Schaffhausen (CH); WEGMANN, Jürgen, 78333 Stockach (DE)
(74) Representative: Patentanwälte Ruff, Wilhelm, Beier, Dauster & Partner mbB
(86) International application number: PCT/EP2011/056910
(87) International publication number: WO 2011/138256

(56) References cited:
- WO-A1-2010/006270
- US-A- 5 350 583
- US-A1- 2003 133 967
- US-A1- 2003 171 824
- US-A1- 2006 136 047

## Description

The invention relates to a process for producing a medical product, in particular a medical implant, more particularly a medical flat or hollow body implant, and also to a medical product, in particular a medical implant, obtained/obtainable by the process.

US 2005/0175659 A1 discloses crosslinked plates of collagen which are prepared from collagen suspensions by lyophilisation and subsequent crosslinking. The collagen plates produced have a porous structure and hence the collagen plates have only limited utility for medical applications requiring enhanced dimensional stability and/or imperviousness to liquids.

US 2006/0136047 A1 discloses multiply laminated tubes whose lumens are lined with a layer of juvenile submucosa. The juvenile submucosa is recovered from natural intestines.

To produce the tubes, the submucosa is grown on a carrier rod. For this, the submucosa can already be provided in its natural tubular form. To construct the tubes, further layers are applied to the carrier rod which are interconnectable using fibrin adhesive or a crosslinking step for example. However, the recovery of natural intestinal submucosa makes the production of the tubes comparatively costly and complicated. Owing to the naturally predetermined diameter of juvenile intestines, moreover, it is not possible to realize just any desired tubular diameter.

US 6,334,872 B1 describes two- or three-ply collagen tubes, the individual plies of which have different properties and are made of different collagens. The inner ply of the collagen tubes is made of acid-extracted collagen for instance. The inner ply also has a smooth surface, resulting in a reduced risk of thrombosis. The outer ply is recovered from intestinal tissue, fascia lata or dura mater of a mammal, and is primarily responsible for the stability of the tubes. The tube shape is produced using suture, stapler or fibrin adhesive. However, these fixing elements required to produce the tubular shape represent an increased introduction of foreign material into the body of a patient. And the production process is made complicated and costly as a result.

US 7,615,063 B2 describes tubular collagen structures for nerve track regeneration. The lumen of the tubular structures is endowed with a sponge material which contains channels for nerve track regeneration. The tube shape is produced by extrusion or spinning of an aqueous collagen solution onto a carrier rod and hence again by a comparatively complicated technology.

US 7,338,517 reveals a collagen tube, the collagen fibres or fibrils of which are aligned in a helical pattern along the longitudinal axis of the tube. Production is by extrusion of a collagen dispersion in a counterrotating extruder. The helical alignment of the fibres or fibrils is defined by the speed of movement of the receiving yarn roller and also the speed of extrusion of the collagen dispersion. The process is likewise comparatively complicated and costly. Another disadvantage is that the tubes obtained have but limited stability in storage. To maintain their tubular structure, the tubes are exposed to a gas stream while they are immersed in a water bath.

US 6,589,257 B1 discloses a tube constructed with three layers which is usable as an artificial nerve guide track in particular. The inner and outer layers are made of collagen or gelatine. The middle layer consists of a net of an absorbable material such as polyglycolic acid for example. The lumen of the tube contains collagen fibres. The cavities between the collagen fibres are said to enable the ingrowth of nerve tracks. The disadvantage here is the use of a net material for producing the tube, which firstly complicates the production process and secondly introduces additional foreign material into the body of a patient.

US 6,461,629 B1 likewise describes a collagen tube for nerve regeneration. The tube is produced by proceeding from a collagen solution. The collagen solution for producing the tube is injected into a cylindrical device having an inner mandrel for producing a lumen. fibrillogenesis is effected by drying the solution while the fibres can also be aligned by applying a magnetic field.

US 2003/133967 A1 describes a multilayer material with improved properties for use in medicine for reconstruction of tissue or organs comprising at least two layers and a multilayer construct.

In view of the known prior art, then, the problem addressed by the present invention is that of providing a very simple, uncomplicated and more particularly universal process for producing medical products, in particular medical implants. The products, in particular implants, produced shall be very shape stable and more particularly have a structure which is substantially impervious to liquids, more particularly water and bodily fluids.

This problem is solved according to the present invention as defined by the appended claims and in particular by a process for producing a medical product, in particular a flat (two-dimensional) or hollow medical product, more particularly a medical implant, preferably a medical flat or hollow body implant, wherein shaped bodies (formed bodies) are facially, two-dimensionally or sheetlike interconnected by means of a solvent.

A liquid-impervious or -impermeable structure is to be understood in the context of the present invention as meaning a structure whose imperviousness to liquids, more particularly water and/or bodily fluids, was evidenced using customary measuring methods known to a person skilled in the art, for example using the Wesolowski method.

An interconnection for the purposes of the present invention shall also more particularly comprehend an interadherance or a lamination, and for the purposes of the present invention it is possible for the interconnection, more particularly interadherance or lamination, not to be brought about using adhesives such as fibrin adhesives or cyanoacrylate adhesives for example. In other words, the shaped bodies may be laminated or laminally interconnected, preferably without the usage of adhesives.

The interconnection for the purposes of the present invention may be more particularly a chemical and/or physical interconnection. More particularly, the interconnection can be based on adhesive forces or molecular interactions such as for example hydrogen bonds and/or Van der Waals forces between the shaped bodies.

Surprisingly, the use of a solvent, preferably water, is sufficient to interadhere shaped bodies facially, preferably laminatelike, to form a shape-stable product, in particular implant, having a preferably substantially liquid-impervious structure.

The product, in particular implant, preferably has a structure impervious to water and/or bodily fluids, for example blood, lymph, cerebrospinal fluid, wound exudate or the like.

In an example, shaped bodies having a structure permeable to liquid, more particularly water and/or bodily fluids, are used for the facial interconnection.

Facial interconnection is preferably made using porous and preferably openly porous shaped bodies. The shaped bodies can have pores having a diameter between 10 and 500 µm, more particularly 20 and 350 µm and preferably 50 and 250 µm.

More particularly, shaped bodies can be used for the facial interconnection that have a foam-, sponge- and/or membranelike structure.

In a further embodiment, the facial interconnection is effected using fibrelike or fibre-shaped shaped bodies. It is preferable for the shaped bodies and more particularly at least some of the shaped bodies to have an irregular or randomized fibrous structure.

In a particularly preferred embodiment, the facial interconnection is made using shaped bodies having a nonwovenlike configuration and more particularly using shaped bodies present as a fibrous nonwoven web or as a bonded fibrous nonwoven web fabric. Shaped bodies having a nonwovenlike configuration may, in addition to fibrous structural fractions, also include other structural fractions, for example leaf- or tull-like structural fractions. Shaped bodies configured as a fibrous nonwoven web or as a bonded fibrous nonwoven web fabric are preferably present as fibre webs, more particularly as purely fibre webs.

In principle, the product, in particular implant, can be produced using shaped bodies having different structures, for example as described in the preceding embodiments. Preferably, however, the shaped bodies used for producing the product, in particular implant, have the same structure.

According to an example, the shaped bodies are selected from the group comprising sponges, foams, membranes, nonwovens or non-woven fabrics, felts, hybrid structures thereof and combinations thereof. Hybrid structures according to the present invention preferably exhibit characteristics of different structures, in particular of structures as mentioned in this paragraph and/or in one of the preceding paragraphs. For example, a hybrid structure according to the present invention may have characteristics of a membrane and of a non-woven fabric.

The shaped bodies provided for producing the product, in particular implant, may be obtained using moulding processes which are per se familiar to a person skilled in the art. For example, the shaped bodies are obtainable using extrusion, more particularly strand extrusion, fibre extrusion or film/sheet extrusion, spinning, more particularly fibre spinning, pressing, more particularly hot pressing, stamping, more particularly microstamping, embossing, rolling, casting, more particularly injection moulding, or blow moulding, more particularly extrusion blow moulding.

Alternatively, the shaped bodies may be also obtainable by a chemical process, for example via sedimentation, precipitation or crystallization, more particularly from a solution, and subsequent drying.

It may be further conceivable according to the present invention for some of the shaped bodies used for producing the product, in particular implant, to be obtained by a moulding process and for the other shaped bodies to be obtained by a chemical process.

In a further example, shaped bodies are facially interconnected which are not obtained via drying processes specific to a porous structure. The facial interconnection is preferably effected using lyophilized (freeze-dried) shaped bodies. Lyophilisation may be envisioned to provide shaped bodies for the facial interconnection which are porous and more particularly fibre shaped and preferably nonwovenlike. Lyophilized shaped bodies are preferably obtained by subjecting liquid dispersions, solutions and/or suspensions, preferably aqueous dispersions, aqueous solutions and/or aqueous suspensions, containing one or more shaped body materials to lyophilisation (freeze drying).

In a further preferred embodiment, sheetlike and preferably flat shaped bodies are used for producing the product, in particular implant. It is preferable, in other words, for the shaped bodies - more particularly at least some of the shaped bodies - to be flat bodies or sheet bodies. Suitable shaped bodies are selectable for example from the group comprising individual layers or plies, discs, strips, plates, foils, films, sheets, membranes, gels, more particularly hydrogels, foams, sponges, bands, tapes, ligaments and combinations thereof.

In an example, the sheetlike interconnection is effected using shaped bodies present in the form of individual plies or layers and more particularly in the form of plates. It is more particularly advantageous therein to interconnect shaped bodies configured as nonwovenlike individual plies or layers and more particularly nonwovenlike plates.

Another example of the process for producing the product, in particular implant, utilizes shaped bodies particularly able to perform a reinforcing function in the final product, in particular implant. Suitable shaped bodies can have a membranelike configuration and are preferably recovered from natural membranes, for example the pericardial membrane.

In one possible example, the shaped bodies and more particularly at least some of the shaped bodies are present as particles or particulate shaped bodies, for example as powder, granules or the like.

The shaped bodies in a further example are dry, dried and/or swollen. For example, the shaped bodies can be swollen in water or in an aqueous medium such as an aqueous solution for example,

According to an example, it is further possible in principle for the shaped bodies and more particularly at least some of the shaped bodies to be individual fibres or threads.

The shaped bodies may in principle have any desired geometries, sizes and/or thicknesses. More particularly, the shaped bodies can have a spherical, spheroidal, circle-shaped and/or oval-shaped configuration and/or have a polygonal, for example a triangular, quadrangular, more particular square and/or rectangular, pentagonal and/or hexagonal, shape.

The shaped bodies preferably include at least one, more particularly exactly one, medically compatible material or a medically compatible mixture of materials, for example a mixture of two, three, four, etc different medically compatible materials, or are preferably made of such a material or of such a mixture of materials.

A medically compatible material for the purposes of the present invention is a material which is generally recognized as safe by physicians and, especially after implantation in the body of a patient, causes no or only medically insignificant damage to health, more particularly tissue.

Preferably, the medically compatible material and the medically compatible mixture of materials are a polymer and a polymer mixture, in particular blend, respectively. The polymer may be more particularly a copolymer, for example a random copolymer and/or block copolymer.

A copolymer for the purposes of an embodiment is a polymer composed of at least two different monomeric units. Accordingly, the shaped bodies envisioned according to the present invention and more particularly at least some of the shaped bodies may also include or be made of a ter- or tetrapolymer.

The medically compatible shaped body material is preferably of xenogenic and more particularly porcine, bovine and/or equine origin. The use of bovine and/or equine materials for producing the shaped bodies is particularly preferred.

In principle, the shaped bodies may also include or be made of a recombinantly produced material.

In a preferred embodiment, the shaped bodies and more particularly at least some of the shaped bodies include a so-called biopolymer, i.e. a naturally occurring polymer, or a polymer recovered or isolated from natural sources, more particularly animal tissues, and optionally further processed, and/or a synthetic biopolymer. More particularly, the shaped bodies and more particularly at least some of the shaped bodies may be made of a biopolymer or a synthetic, i.e. manufactured, biopolymer.

Preferably, the shaped bodies and more particularly at least some of the shaped bodies include or are made of a medically compatible material from the group comprising peptides, proteins, more particularly extracellular proteins or connective tissue proteins, preferably fibre-shaped or fibrelike proteins, oligopeptides, polypeptides such as for example polylysine, polyaminoacids, more particularly synthetic polyaminoacids, polysaccharides, salts thereof and mixtures thereof. Suitable polysaccharides are more particularly selected from the group comprising oxidized polysaccharides, for example polysaccharides bearing aldehyde groups, alkylcelluloses, hydroxyalkylcelluloses, carboxyalkylcelluloses, amino-bearing polysaccharides, mucopolysaccharides or glycosaminoglycans, salts thereof and mixtures thereof. The peptides, oligopeptides, polypeptides and polyamino acids mentioned in this paragraph can be constructed of one type of amino acid and/or different amino acids.

The shaped bodies and more particularly at least some of the shaped bodies preferably include or are made of a medically compatible material selected from the group comprising collagen, gelatin, elastin, fibronectin, reticulin, albumin, starch, aldehydic starch, amylose, amylopectin, dextran, dextran aldehyde, cellulose, oxidized cellulose, methylcellulose, hydroxymethylcellulose, hydroxyethylcellulose, hydroxyethylmethylcellulose, hydroxypropylmethylcellulose, hydroxybutylmethylcellulose, carboxymethylcellulose, chitin, chitosan, hyaluronic acid, chondroitin 4-sulphate, chondroitin 6-sulphate, dermatan sulphate, keratan sulphate, heparin, heparan sulphate, derivatives thereof, salts thereof and mixtures thereof.

It is particularly preferable for the shaped bodies and more particularly at least some of the shaped bodies to include or be made of collagen. The collagen is preferably a fibre-forming or fibrillary collagen. Preference is given to collagen of type I, II, III, IV, V, VI and/or XI. The collagen may be present as native insoluble collagen in particular. A native insoluble collagen for the purposes of the present invention is a collagen which in a chemically unmodified state is insoluble in an aqueous alkaline solution, an aqueous acidic solution or some other aqueous inorganic solution of a salt.

In a further example, the shaped bodies and more particularly at least some of the shaped bodies include or are made of a synthetic polymer preferably selected from the group comprising polyvinyl alcohol, polyvinyl acetate, polyethylene glycol, polyvinylpyrrolidone, polyethyleneimine, polyhydroxyalkanoates, poly-ε-caprolactone, poly-trimethylene carbonate, poly-para-dioxanone, copolymers thereof and mixtures, in particular blends, thereof. Preferred polyhydroxyalkanoates may be selected from the group comprising polyglycolide, polylactide, polyhydroxybutyrates, poly-3-hydroxybutyrate, poly-4-hydroxybutyrate, copolymers thereof and mixtures, in particular blends, thereof.

In principle, the shaped bodies used for producing the product, in particular implant, may include or be made of different materials, in particular mixtures of materials. However, it can be preferable according to the present invention, particularly from process-engineering and economic viewpoints, for the shaped bodies each to include or be made of the same material or the same mixture of materials. With regard to advantageous materials and mixtures of materials, reference is made to the materials and mixtures of the materials described in the preceding embodiments.

In addition to the medically compatible materials described in the preceding embodiments, the shaped bodies may additionally also contain additives, for example pharmaceutically active substances, salts or the like. With regard to suitable additives reference is made to the description hereinbelow, particularly in connection with the solvent contemplated according to the present invention.

In a further example, shaped bodies used for the facial interconnection have surfaces with a partial or complete coating. Preferably, only one side and more preferably only one facial side of the shaped bodies is coated. Useful coating materials include for example the abovementioned biocompatible materials, of which collagen, gelatin, salts thereof or mixtures thereof are particularly preferred.

The solvent contemplated according to the present invention may be a single solvent or alternatively a solvent mixture.

In a particularly preferred embodiment, the facial interconnection of the shaped bodies is effected using water as solvent. Surprisingly, even pure water turns out to be completely sufficient as interconnecting agent to interconnect the shaped bodies facially to form a shape-stable, more particularly tension- and compression-resistant, product, in particular implant, which moreover preferably has a substantially liquid-impervious product structure, in particular implant structure.

Alternatively or additionally, organic solvents, preferably hydrophilic solvents, can be used for the facial interconnection of the shaped bodies. A hydrophilic solvent for the purposes of the present invention is an organic solvent which is soluble in water, preferably in any proportion. In other words, the term "hydrophilic solvent" may also encompass organic solvents which are only partially soluble in water. Polar protic and/or polar aprotic solvents are preferred in principle. Alcohols, more particularly low molecular weight, preferably water-soluble, alcohols are particularly preferred. The alcohols in question can also be in particular diols and/or triols. Preferred solvents further include ketones, more particularly low molecular weight or short-chain ketones.

Preference is given to using a solvent selected from the group comprising water, methanol, ethanol, propanol, isopropanol, acetone, DMSO and mixtures thereof.

It is particularly advantageous for the solvent contemplated according to the present invention to be free of additives or auxiliaries, more particularly free of adhesives such as fibrin adhesives or cyanoacrylate adhesives for example. It may be preferable, in other words, for the shaped bodies to be facially interconnected using a pure solvent, preferably using pure water. The solvent used for this can be in its customary degrees of purity. Alternatively, a pure solvent mixture, or a solvent mixture which is free of adhesives, can also be used for facially interconnecting the shaped bodies.

It may further be envisaged for the solvent to be used for the facial interconnection of the shaped bodies in the form of a dispersion, solution or suspension, more particularly aqueous dispersion, aqueous solution or aqueous suspension.

It may be particularly reasonable from medical viewpoints to endow the product, in particular implant, with particular properties. For this, it is particularly advantageous to add appropriate additives to the solvent. Preferably, the solvent contains additives selected from the group comprising inorganic salts, more particularly alkali metal and/or alkaline earth metal halides, organic salts, more particularly fatty acid salts, medical/medicinal/pharmaceutical substances, more particularly antimicrobial substances, preferably antibiotics such as for example gentamycin, polyhexamethylenebiguanide, triclosan, copper, silver, gold or salts thereof, disinfectants, anti-inflammatory substances, wound healing promoter substances, analgesic substances, odour control substances, cellular growth factors, cellular differentiation factors, cellular recruitment factors, cellular adhesion factors, cell binding sequences, plasticizers such as for example glycerol and combinations thereof. Additizing the solvent with inorganic salts is a way to increase the conductivity of the implant for example. This is particularly advantageous for example to perform anastomoses or vessel sealing using the thermal fusion technique (TFT) in particular. Additizing the solvent with water-soluble salts has the further advantage that these salts can be dissolved out of the product, in particular implant, structure in the body of a patient. This creates holes or pores in the product, in particular implant, structure to speed the tissue regeneration and/or remodelling by the ingrowth of endogenous cells for example. Alternatively, these salts can also be specifically leached and/or dissolved out of the product, in particular implant, prior to implantation.

Alternatively, the product, in particular implant, can also be additized after it has been produced, more particularly after the facial interconnection of the shaped bodies. To this end, the product, in particular implant, can be endowed with the desired additives as part of a post-treatment for example. With regard to suitable additives, reference is made to the additives described in the preceding embodiment.

According to the invention, the shaped bodies to be facially interconnected are moistened or wetted with the solvent. To moisten the shaped bodies with the solvent, they can be brushed, sprayed, overpoured with the solvent and/or dipped or immersed into the solvent. The moistening of the shaped bodies can optionally also be repeated. Preferably, the shaped bodies are sprayed with the solvent for facial interconnection.

Solvent-moistened shaped bodies are laid or stacked onto each other, particularly on a carrier, for facial interconnection. Alternatively, the shaped bodies can also be needled together. It is particularly preferable for solvent-moistened shaped bodies configured as flat or sheet bodies, more particularly as individual plies or layers, to be laminated on top of each other. With regard to further features and advantages of the carrier, the observations hereinbelow are referenced in full.

It is particularly preferable to perform a drying operation, more particularly a warm drying operation, to achieve a durable interconnection of the shaped bodies. Preferably, drying is carried out with at least partial and preferably complete elimination of layer or phase boundaries between the shaped bodies. The drying temperature can be very advantageously adapted to the thermal sensitivity of the shaped body materials used. Any drying operation is preferably carried out in a temperature range between 20°C and 80°C, more particularly 25°C and 45°C and preferably 30°C and 40°C.

Alternatively, the shaped bodies can also be dried by lyophilisation (freeze drying). It is a particular advantage that a single drying step is completely sufficient for producing the product, in particular implant.

According to the invention, the shaped bodies are facially interconnected by the solvent-moistened shaped bodies being laid or stacked onto each other and then dried. Multiple interconnecting and more particularly drying steps can be avoided in this way.

To establish adequate contact between the shaped bodies for the facial interconnection, the shaped bodies can be subjected to a force, preferably a pressing force, before and/or during any drying step in particular.

To produce a flat medical product, preferably a flat body implant, for example in the form of a membrane, foil, film, sheet, tape, band or ligament, it is advantageous for shaped bodies in a flat and more particularly planar configuration, more particularly in the form of individual plies or layers, for example plates, to be facially interconnected on a flat and more particularly planar carrier. To further tissue regeneration and/or reconstruction, the product, in particular implant, can be apertured or perforated in a subsequent step. With regard to further features and advantages thereof, the description hereinabove is referenced in full.

To produce a hollow medical product, preferably a hollow body implant, preferably a tubular or tube-shaped implant, the shaped bodies are facially interconnected on a carrier responsible for forming a cavity. To this end, the shaped bodies are preferably mounted on the carrier in a positively locking fashion (form-fit fashion), or the carrier is preferably ensheathed or overwrapped by the shaped bodies in a positively locking fashion (form-fit fashion). The carrier used can be a pin, a mandrel, a mould core, a tube or cylinder. The shape or geometry, more particularly the cross-section, of the carrier determines the shape or geometry, more particularly the cross-section, of the hollow body of the product, preferably implant. The carrier may have for example a circularly round, oval-shaped or polygonal, more particularly triangular, quadrangular, more particularly square or rectangular, pentagonal, hexagonal or star-shaped, cross-section. A circularly round cross-section of the carrier is preferred. The carrier preferably has a tubular or tube-shaped (cylindrical) or conical configuration.

According to the invention, the shaped bodies are placed or stacked onto each other on a carrier responsible for forming a cavity such that mutually opposite ends, more particularly transversal side ends, of the shaped bodies touch, preferably abuttingly. It is preferable for the end of a shaped body to be mounted on the carrier to be mounted abuttingly in respect of the start of a shaped body previously mounted on the carrier. Interface inhomogeneities in the product, in particular implant, structure can be avoided in this way to particular advantage.

Flat products, preferably flat body implants, according to the present invention may also be obtainable from hollow products, in particular from hollow body implants, obtained according to the present invention by moistening the hollow bodies of the hollow medical products, in particular of the hollow body implants, with the solvent contemplated according to the present invention and by the medical products, in particular hollow body implants subsequently being pressed flat and dried.

In principle, any number of shaped bodies can be used to produce the medical product, in particular implant. Preferably the number of shaped bodies facially interconnected is in the range from 2 to 15, more particularly in the range from 2 to 10 and more preferably in the range fro 2 to 5. The number of shaped bodies used for producing the product, in particular implant, is a particularly advantageous way to improve/enhance the shape stability and more particularly imperviousness of the product, in particular implant. However, a particular advantage of the present invention resides in the fact that liquid-impervious products, in particular implants, are obtainable in the facial interconnection of just two shaped bodies.

Although the products, in particular implants, of the present invention are in and of themselves already sufficiently shape-stable and more particularly liquid-impervious, the present invention does make it possible for the medical product, in particular implant, to be crosslinked in a subsequent treatment step for example. Thermal, radiation-induced and/or chemical methods of crosslinking can be used for this. The crosslinking of the medical product, in particular implant, can take place more particularly in the gas phase and/or in a solution. Preferably, the medical product, in particular implant, is crosslinked using chemical crosslinking agents preferably selected from the group comprising formaldehyde, dialdehydes, more particularly glutaraldehyde, carbodiimides, diisocyanates, for example hexamethylene diisocyanate, bifunctional succinimides and combinations thereof.

In an advanced embodiment, the medical product, in particular implant, is crosslinked in the presence of a plasticizer, for example glycerol.

The present invention further provides a medical product, in particular a flat (two-dimensional) or hollow medical product, more particularly a medical implant, preferably a medical flat or hollow body implant, obtained or obtainable via or by means of a facial, two-dimensional or sheetlike interconnection of shaped bodies (formed bodies) which are preferably present in the form of individual plies or layers, using a solvent, preferably to construct or form an essentially liquid-impervious, preferably completely liquid-impervious, product structure, in particular implant structure.

The product, in particular implant, is particularly advantageous in having high dimensional stability, preferably breaking strength and more particularly compressive resistance.

The product, in particular implant, preferably has a structure impervious to water and/or bodily fluids.

Preferably, the interconnected shaped body faces, more particularly at least some of the interconnected shaped body faces, do not form any layer or phase boundaries in the final product, in particular implant. In other words, it may be preferable according to the present invention for the product, in particular implant, not to have any layer or phase boundaries originating in the facial interconnection of the shaped bodies. More particularly, the product, in particular implant, can be completely free of layer or phase boundaries.

In an example, the product, in particular implant, is free of securing or fixing means, more particularly adhesives, stitches, staples or the like.

It is further preferable for the product, in particular implant, to have a linear breaking force or strength of above 2 N, more particularly above 5 N and preferably above 10 N.

Further, the product, in particular implant, may have a linear tensile force from 2 to 400 N, in particular from 5 to 300 N and preferably from 10 to 200 N.

The product, in particular implant, may further have a radial breaking force or strength of above 2 N, more particularly above 5 N and preferably above 10 N.

Further, the product, in particular implant, may have a radial breaking force from 2 to 400 N, in particular from 5 to 300 N and preferably from 10 to 200 N.

More particularly, the product, in particular implant, may have a thread pull-out force or strength of above 2 N, more particularly above 5 N and preferably above 10 N.

Further, the product, in particular implant, may have a thread pull-out force from 2 to 400 N, in particular from 5 to 300 N and preferably from 10 to 200 N.

In an especially preferred embodiment, the medial product, in particular the medical implant is present as a laminate product, in particular laminate implant.

In a further example, the product, in particular implant, is configured as a flat body, preferably as a membrane, tape, band, ligament, foil, film or sheet. It may further be preferable in this embodiment for the product, in particular implant, to have a structured or profiled surface or alternatively apertures, perforations or the like to promote the ingrowth of body tissue and body cells into the product, in particular implant, and hence generally the regeneration and/or reconstruction of tissue. More particularly, aperturing or perforating a product, in particular an implant, configured as a flat body can be used to provide meshlike structures in the manner of hernia, prolapse or incontinence meshes in particular. In other words, the products, in particular implants, of the present invention can also be configured as hernia, prolapse or incontinence products, in particular implants.

In another example, the product, in particular implant, is configured as a hollow body, more particularly as a tube, as a cone or as a ring. A ring-shaped product, in particular implant, may be obtainable to particular advantage by trimming a tube-shaped product, in particular implant, obtained according to the present invention.

As mentioned more than once, the product, in particular implant, may preferably be a tubular or tube-shaped (substantially cylindrical) product, in particular implant. Particular preference is given to a tube-shaped product, in particular implant, based on a protein, more particularly an extracellular protein or connective tissue protein, preferably of collagen (collagen tube).

The product, in particular implant, is sterilizable in particular in that the advantageous properties of the product, in particular implant, are particularly advantageously not impaired by a sterilizing operation. It can therefore be preferable according to the present invention for the product, in particular implant, to be in a sterilized state. The product, in particular implant, may be sterilized using γ-sterilization or ethylene oxide sterilization for example.

The medical product, in particular implant, is preferably useful as a surgical implant. The product, in particular implant, of the present invention is preferably provided for reconstruction, augmentation, sealing and/or fusion of human and/or animal tissues, more particularly hollow organs and/or vessels, preferably blood vessels. To carry out tissue fusions, more particularly anastomoses, it is preferable to use tubular and/or ring-shaped products, in particular implants. To carry out tissue sealing, more particularly to seal off organ leakages, for example urethra and/or lung leakages, or other wound leakages, products, in particular implants, configured as membranes, tapes, bands, ligaments, foils, films or sheets can be used for example.

Further, the medical product may be useful for topological applications. For example, the medical product may be selected from the group comprising hamostatic device, patch, non-woven fabric, fibrous web, felt, and wound dressing.

In a further example, the medical product is a hollow medical product, in particular for ensheathing limbs, such as fingers.

The product, in particular implant, of the present invention may further also be useful as a tissue guide reel, more particularly for nerve tissue or nerve tracks.

With respect to further features and advantages of the medical product, in particular medical implant, reference is made in its entirety to the features and advantages of the process according to the present invention.

The advantages of the invention will now be summarised once more:
The present invention is based on the surprising realization that the interconnecting force of a solvent, pure water in particular, is sufficient to facially interconnect individual shaped bodies to form shape-stable products, in particular implants. To achieve shape stability which is adequate from medical viewpoints, it is especially not necessary to subject the products, in particular implants, to a subsequent crosslinking step. Nor is the use of adhesives or adhesive additives necessary to obtain shape-stable products, in particular implants. Tedious and laborious polymerization/curing periods can be avoided as a result. Nor are fixing aids such as for example sutures, staplers or the like needed to carry out the process of the present invention. This is particularly advantageous in reducing the input of foreign material in the body of a patient. Altogether, the process of the present invention represents a significantly simplified and more particularly more economical way to produce patient-compatible products, in particular implants, compared with the production processes known from the prior art.

A further advantage of the process according to the present invention concerns its universal character. The process according to the present invention is able to provide in principle products, in particular implants, having a very wide variety of shapes, geometries and/or thicknesses. The process according to the present invention is useful not only for producing hollow medical products, in particular hollow body implants, more particularly hose- or tube-shaped implants, more particularly in any desired diameters, but also for producing flat medical products, particularly flat body implants, preferably medical bands, tapes, ligaments, foils, films or sheet, which may optionally be apertured and/or perforated in order that tissue regeneration and/or reconstruction may be furthered in vivo.

As mentioned more than once, the products, in particular implants, of the present invention are notable for excellent dimensional stability. This excellent dimensional stability manifests particularly in a breaking strength and particularly compressive strength which are optimal for medical purposes. The products, in particular implants, are thereby readily able to withstand the forces typically occurring in the body of a patient. A further advantage concerns the preferably liquid-impervious structure of the product, in particular implant. A diversity of possible medical uses arise from it. The products, in particular implants, of the present invention are particularly useful for carrying out vessel and/or wound sealing. They are also usable as a cell tissue guide rail, for example for nerve tissue. Further features, details and advantages of the present invention will be apparent from the following description of preferred embodiments with reference to examples combined with the dependent claims. Features can each be actualized singly or in combination with each or one another. The examples merely serve to illustrate the invention and are not in any way to be understood as limiting.

### Example 1

### Producing a coliagen tube from two collagen nonwovens

Two collagen nonwovens, each having a basis weight of about 125 g/m² and a size of 40 x 16 cm (640 cm²), were moistened with water using a spray applicator. Then, a cylindrical carrier form (25 mm outer diameter and 40 cm length) was ensheathed with one of the two moistened collagen nonwovens in a positively locking fashion. This was followed by a second overwrapping of the carrier form with the other moistened collagen nonwoven (covering ply). To avoid interface inhomogeneities, the end of the second collagen nonwoven was laminated abuttingly onto the beginning of the first wrap. The wrapped collagen nonwovens were then dried at 37°C for 24 hours and subsequently removed from the carrier form. The collagen tube obtained in a length of 40 cm had a basis weight of about 250 g/m².

### Example 2

### Collagen tube from four collagen nonwovens with subsequent crosslinking

Four collagen nonwovens having a basis weight of about 125 g/m² and a size of 40 x 32 cm (1280 cm²) were moistened with water using a spray applicator. Then, a cylindrical carrier form (25 mm outer diameter and 40 cm length) was ensheathed with one of the moistened collagen nonwovens in a positively locking manner. This was followed by three further overwrappings of the carrier form with the other moistened collagen nonwovens (covering plies). To avoid interface inhomogeneities, the end of the most recently applied collagen nonwoven was laminated abuttingly onto the beginning of the previously wrapped collagen non-woven. The wrapped collagen nonwovens were subsequently dried at 37°C for 24 hours and then removed from the carrier form. The collagen tube obtained in a length of 40 cm had a basis weight of about 500 g/m². To enhance its stability, the collagen tube was dipped for 6 hours into a crosslinking solution of 75 g of isopropanol, 8.04 g of deionized water, 16.87 g of glycerol and 90.82 µl of hexamethylene diisocyanate (HMDI). Subsequently, unbound hexamethylene diisocyanate was rinsed off and the crosslinked collagen tube was again dried at 37 °C.

### Example 3

### Producing a collagen tube from three thin collagen nonwovens with subsequent crosslinking

Three thin collagen nonwovens having a basis weight of about 85 g/m² and a size of 40 x 24 cm (960 cm²) were moistened with water using a spray applicator. Thereafter, a cylindrically shaped carrier form (25 mm outer diameter and 40 cm length) was overwrapped with one of the moistened collagen nonwovens in a positively locking fashion. This was followed by two further overwrappings with the other two moistened collagen nonwovens (covering plies). To avoid interface inhomogeneities, the end of the most recently wrapped collagen web was laminated abuttingly onto the beginning of the previously wrapped collagen non-woven. The wrapped collagen nonwovens were then dried at 37°C for 24 hours. After drying, the wrapped collagen nonwovens were removed from the carrier form to obtain a collagen tube 40 cm in length and having a basis weight of about 255 g/m². To enhance its stability, the collagen tube was dipped for 6 hours into a crosslinking solution of 75 g of isopropanol, 8.04 g of deionized water, 16.87 g of glycerol and 90.82 µl of hexamethylene diisocyanate (HMDI). Unbound hexamethylene diisocyanate was subsequently rinsed off and the collagen tube again dried at 37 °C.

The specimens produced according to Examples 1 to 3 were each subjected to a γ sterilization process and an ethylene oxide sterilization process after drying. The specimens did not show any changes in the sterilized state, especially no disadvantageous changes whatsoever.

### Example 4

### 4. Characterizing the collagen tubes produced in Examples 1 to 3

### 4.1 Determining the linear breaking force

The collagen tubes produced in Examples 1 to 3 were each cut to excise 10 mm wide and 50 mm long strips as sample materials. The strips were clamped in the dry state, or after swelling in aqueous buffer solution (pH 7.4), in a tensile tester to determine the breaking force of the strips. The results obtained are shown in Tables 1 and 2.

### 4.2 Determining the radial breaking force

The collagen tubes produced in Examples 1 to 3 were cut to excise rings 10 mm in width. Some of the rings were threaded in the dry state onto the holders of a tensile tester to determine the breaking force. The remaining rings were first swollen in an aqueous buffer solution (pH 7.4) before being threaded onto the holders of the tensile tester. The results are likewise reproduced in Tables 1 and 2.

### 4.3 Determining the thread pull-out force

The collagen tubes produced in Examples 1 to 3 were cut to excise sample pieces measuring 4 x 2 cm. Some of the sample pieces were swollen in an aqueous buffer solution (pH 7.4). The dry and swollen sample pieces were pierced from above at 1 cm from the lateral edge with a monofil suture of USP 2-0 size and fixed into the upper jaw of a tensile tester. The lower end of the sample pieces was fixed in the lower jaw. The subsequent measurement was used to determine the maximum force until complete pull-out of the thread from the sample body. The results are likewise reproduced in Tables 1 and 2.

**Table 1**

| Sample | Linear breaking force dry | Radial breaking force dry | Thread pull-out force dry |
|---|---|---|---|
| Example 1 | 9.5 N | 12.6 N | 5.4 N |
| Example 2 | 22.1 N | 28.1 N | 23.4 N |
| Example 3 | 12.7 N | 14.3 N | 8.9 N |

**Table 2**

| Sample | Linear breaking force after swelling | Radial breaking force after swelling | Thread pull-out force after swelling |
|---|---|---|---|
| Example 1 | 3.9 N | 8.9 N | 3.6 N |
| Example 2 | 10.1 N | 13.7 N | 11.3 N |
| Example 3 | 4.4 N | 9.3 N | 4.9 N |

### 4.4 Determining imperviousness under static pressure

The collagen tubes produced in Examples 1 to 3 were each cut to tubes having a shorter length. The latter were used as test specimens and sealed on one side, filled with deionized water and finally exposed to compressed air at 120 mmHg for 30 minutes. Imperviousness was checked thereafter. The result is shown in Table 3.

### 4.5 Determining imperviousness under dynamic pressure

The collagen tubes produced in Examples 1 to 3 were each cut into tubes of smaller length. The latter were used as test specimens and fitted both sides with adaptors for the imperviousness tester and placed into a closed container filled with deionized water. The adaptors were coupled to a pump system and exposed to a pulsating pressure of 180/90 mmHg at a frequency of 70 per minute for 60 minutes. This was followed by an inspection for imperviousness through displaced water at the overflow. The results are likewise shown in Table 3.

### 4.6 Determination of Wesolowski imperviousness

The collagen tubes produced in Examples 1 to 3 were trimmed to sample pieces measuring 2.5 x 2.5 cm, which were inserted into a clamping device. After activation of the system, the sample pieces were each loaded with a pressure of a water column 166 cm in length (corresponds to 122 mmHg) on an area of 1 cm². Imperviousness was determined by the amount of water permeating through the sample during one hour. The results are likewise shown in Table 3.

**Table 3**

| Sample | Static imperviousness | Dynamic imperviousness | Wesolowski |
|---|---|---|---|
| Example1 | impervious | impervious | impervious |
| Example2 | impervious | impervious | impervious |
| Example3 | impervious | impervious | impervious |

### Example 5

### Producing a collagen membrane from three collagen nonwovens with subsequent crosslinking

Three collagen nonwovens having a basis weight of about 125 g/m² and a size of 20 cm x 20 cm (400 cm²) were moistened with water using a spray applicator. Then, a planar carrier plate had one of the moistened collagen nonwovens laid onto it in a positively locking fashion. This was followed by two further superpositions of the carrier form with the other moistened collagen nonwovens (covering plies). To avoid inhomogeneities, the collagen nonwoven applied most recently was congruently overlaminated with the following collagen nonwoven. The laminated collagen nonwovens were then dried at 37°C during 24 hours and subsequently removed from the carrier plate. The collagen membrane obtained, 20 cm x 20 cm in size, had a basis weight of about 375 g/m². To enhance its stability, the collagen membrane was dipped for 6 hours into a crosslinking solution of 75 g of isopropanol, 8.04 g of deionized water, 16.87 g of glycerol and 90.82 µl of hexamethylene diisocyanate. Subsequently, unbound hexamethylene diisocyanate was rinsed off and the crosslinked collagen membrane was again dried at 37°C.

## Claims

1. Process for producing a medical product, in particular medical implant, wherein shaped bodies are facially interconnected by means of a solvent, the shaped bodies to be interconnected are moistened with the solvent, wherein a hollow body product is produced by facially interconnecting the shaped bodies on a carrier responsible for forming a cavity, **characterized in that** the shaped bodies are placed onto each other on the carrier, wherein the end of a shaped body to be mounted on the carrier is mounted abuttingly in respect of the start of a shaped body previously mounted on the carrier, moistening the hollow body product with the solvent, and subsequently pressing the hollow body product flat and drying it.

2. Process according to Claim 1, **characterized in that** shaped bodies used for the facial interconnection have a porous, more particularly an openly porous, structure, the facial interconnection is in particular effected using fibrelike or fibre-shaped shaped bodies, preferably using nonwovenlike shaped bodies.

3. Process according to claim 1 or 2, **characterized in that** the facial interconnection is effected using lyophilized shaped bodies.

4. Process according to any preceding claim, **characterized in that** the facial interconnection is effected using shaped bodies in the form of sheet bodies and more particularly in the form of individual plies or layers.

5. Process according to any preceding claim, **characterized in that** the shaped bodies include or are made of a biopolymer selected from the group comprising polyamino acids, polypeptides, proteins, more particularly extracellular proteins or connective tissue proteins, polysaccharides, more particularly oxidized poly-saccharides and/or mucopolysaccharides or glycosaminoglycans, salts thereof and combinations thereof.

6. Process according to any preceding claim, **characterized in that** water and/or a hydrophilic solvent, more particularly a water-soluble alcohol, is used as solvent.

7. Process according to any preceding claim, **characterized in that** the facial interconnection is effected using purely solvent or purely solvent mixture.

8. Process according to any one of claims 1 to 6, **characterized in that** the solvent is used in the form of a dispersion, solution or suspension.

9. Process according to any one of claims 1 to 6 or 8, **characterized in that** a solvent used for the facial interconnection of the shaped bodies contains additives, preferably selected from the group comprising inorganic salts, more particularly alkali metal and/or alkaline earth metal halides, organic salts, more particularly fatty acid salts, medical or pharmaceutical substances, more particularly antimicrobial substances, preferably antibiotics, disinfectants, anti-inflammatory substances, wound healing promoter substances, analgesic substances, odour control substances, cellular growth factors, cellular differentiation factors, cellular recruitment factors and/or cellular adhesion factors, plasticizers and combinations thereof.

10. Process according to any preceding claim, **characterized in that** the shaped bodies are facially interconnected by drying, more particularly in a temperature range between 20°C and 80°C, more particularly 25°C and 45°C and preferably 30°C and 40°C.

11. Process according to any preceding claim, **characterized in that** a hollow body implant, preferably a tubular implant, is produced by facially interconnecting the shaped bodies on the carrier, preferably on a cylindrically or conically shaped carrier.

12. Process according to any preceding claim, **characterized in that** the product is crosslinked, for example in a subsequent treatment step and preferably in the presence of a plasticizer.

## Patentansprüche

1. Verfahren zur Herstellung eines medizinischen Produkts, insbesondere eines medizinischen Implantats, bei welchem Formkörper mittels eines Lösungsmittels flächig miteinander verbunden werden, die zu verbindenden Formkörper mit dem Lösungsmittel befeuchtet werden, bei dem ein Hohlkörperprodukt durch flächiges Verbinden der Formkörper miteinander auf einem für die Ausbildung eines Hohlraumes verantwortlichen Träger ausgebildet wird, **dadurch gekennzeichnet, dass** die Formkörper auf dem Träger übereinander gelegt werden, wobei das Ende eines auf den Träger anzubringenden Formkörpers auf Stoß auf den Anfang eines zuvor auf den Träger angebrachten Formkörpers aufgebracht wird, das Hohlkörperprodukt mit dem Lösungsmittel befeuchtet wird und anschließend das Hohlkörperprodukt flach gepresst und getrocknet wird.

2. Verfahren nach Anspruch 1, **dadurch gekennzeichnet, dass** zur flächigen Verbindung verwendete Formkörper eine poröse, insbesondere offenporöse, Struktur aufweisen und die flächige Verbindung insbesondere unter Verwendung von faserartigen bzw. faserförmigen Formkörpern, vorzugsweise mit vliesartigen Formkörpern, durchgeführt wird.

3. Verfahren nach Anspruch 1 oder 2, **dadurch gekennzeichnet, dass** die flächige Verbindung mit lyophilisierten Formkörpern vorgenommen wird.

4. Verfahren nach einem der vorhergehenden Ansprüche, **dadurch gekennzeichnet, dass** die flächige Verbindung mit in Form von Flächengebilden, insbesondere mit in Form von einzelnen Lagen bzw. Schichten, vorliegenden Formkörpern durchgeführt wird.

5. Verfahren nach einem der vorhergehenden Ansprüche, **dadurch gekennzeichnet, dass** die Formkörper ein Biopolymer aufweisen oder aus einem Biopolymer hergestellt sind, welches ausgewählt ist aus der Gruppe umfassend Polyaminosäuren, Polypeptide, Proteine, insbesondere extrazelluläre Proteine bzw. Bindegewebsproteine, Polysaccharide, insbesondere oxidierte Polysaccharide und/oder Mucopolysaccharide bzw. Glykosaminoglykane, Salze davon und Kombinationen davon.

6. Verfahren nach einem der vorhergehenden Ansprüche, **dadurch gekennzeichnet, dass** Wasser und/oder ein hydrophiles Lösungsmittel, insbesondere ein wasserlöslicher Alkohol, als Lösungsmittel verwendet wird.

7. Verfahren nach einem der vorhergehenden Ansprüche, **dadurch gekennzeichnet, dass** die flächige Verbindung mit einem reinen Lösungsmittel oder einem reinen Lösungsmittelgemisch vorgenommen wird.

8. Verfahren nach einem der Ansprüche 1 bis 6, **dadurch gekennzeichnet, dass** das Lösungsmittel in Form einer Dispersion, Lösung oder Suspension verwendet wird.

9. Verfahren nach einem der Ansprüche 1 bis 6 oder 8, **dadurch gekennzeichnet, dass** zur flächigen Verbindung der Formkörper ein Lösungsmittel verwendet wird, welches Additive enthält, vorzugsweise ausgewählt aus der Gruppe umfassend anorganische Salze, insbesondere Alkalimetall- und/oder Erdalkalimetallhalogenide, organische Salze, insbesondere Fettsäuresalze, medizinische bzw. pharmazeutische Substanzen, insbesondere antimikrobielle Substanzen, bevorzugt Antibiotika, Desinfektionsmittel, entzündungshemmende Substanzen, wundheilungsfördernde Substanzen, schmerzlindernde Substanzen, geruchsbekämpfende Substanzen, zelluläre Wachstumsfaktoren, zelluläre Differenzierungsfaktoren, zelluläre Rekrutierungsfaktoren und/oder zelluläre Adhäsionsfaktoren, Weichmacher und Kombinationen davon.

10. Verfahren nach einem der vorhergehenden Ansprüche, **dadurch gekennzeichnet, dass** zur flächigen Verbindung der Formkörper eine Trocknung, insbesondere in einem Temperaturbereich zwischen 20 °C und 80 °C, insbesondere 25 °C und 45 °C, bevorzugt 30 °C und 40 °C, durchgeführt wird.

11. Verfahren nach einem der vorhergehenden Ansprüche, **dadurch gekennzeichnet, dass** ein Hohlkörperimplantat, vorzugsweise ein tubuläres Implantat, durch flächiges Verbinden der Formkörper miteinander auf dem Träger, vorzugsweise auf einem zylindrisch oder konisch geformten Träger, ausgebildet wird.

12. Verfahren nach einem der vorhergehenden Ansprüche, **dadurch gekennzeichnet, dass** das Produkt, beispielsweise in einem nachträglichen Behandlungsschritt und vorzugsweise in Gegenwart eines Weichmachers, vernetzt wird.

## Revendications

1. Procédé pour produire un produit médical, en particulier un implant médical, dans lequel des corps façonnés sont interconnectés au niveau facial au moyen d'un solvant, les corps façonnés devant être interconnectés sont humidifiés par le solvant, dans lequel un produit de type corps creux est produit par interconnexion au niveau facial des corps façonnés sur un support responsable de la formation d'une cavité, **caractérisé en ce que** les corps façonnés sont placés les uns sur les autres sur le support, dans lequel la fin d'un corps façonné devant être monté sur le support est montée en butée par rapport au début d'un corps façonné préalablement monté sur le support, le produit de type corps creux est humidifié par le solvant, et ensuite le produit de type corps creux est pressé jusqu'à devenir plat et est séché.

2. Procédé selon la revendication 1, **caractérisé en ce que** les corps façonnés utilisés pour l'interconnexion faciale ont une structure poreuse, plus particulièrement à pores ouverts, et l'interconnexion faciale est en particulier réalisée par utilisation de corps façonnés en forme de fibres ou analogues à des fibres, de préférence par utilisation de corps façonnés de type non-tissé.

3. Procédé selon la revendication 1 ou 2, **caractérisé en ce que** l'interconnexion faciale est effectuée par utilisation de corps façonnés lyophilisés.

4. Procédé selon l'une quelconque des revendications précédentes, **caractérisé en ce que** l'interconnexion faciale est effectuée par utilisation de corps façonnés en forme de corps en feuilles et plus particulièrement en forme de couches ou plis individuels.

5. Procédé selon l'une quelconque des revendications précédentes, **caractérisé en ce que** les corps façonnés comprennent ou sont constitués d'un biopolymère choisi dans l'ensemble constitué par les poly(acides aminés), les polypeptides, les protéines, plus particulièrement les protéines extracellulaires ou les protéines de tissu conjonctif, les polysaccharides, plus particulièrement les polysaccharides oxydés et/ou les mucopolysaccharides ou les glycosaminoglycanes, leurs sels, et leurs combinaisons.

6. Procédé selon l'une quelconque des revendications précédentes, **caractérisé en ce que** de l'eau et/ou un solvant hydrophile, plus particulièrement un alcool soluble dans l'eau, sont utilisés en tant que solvant.

7. Procédé selon l'une quelconque des revendications précédentes, **caractérisé en ce que** l'interconnexion faciale est effectuée par utilisation uniquement de solvant ou uniquement d'un mélange solvant.

8. Procédé selon l'une quelconque des revendications 1 à 6, **caractérisé en ce que** le solvant est utilisé sous la forme d'une dispersion, solution ou suspension.

9. Procédé selon l'une quelconque des revendications 1 à 6 ou 8, **caractérisé en ce que** le solvant utilisé pour la connexion faciale des corps façonnés contient des additifs, de préférence choisis dans l'ensemble comprenant les sels inorganiques, plus particulièrement les halogénures de métal alcalin et/ou de métal alcalino-terreux, les sels organiques, plus particulièrement les sels d'acide gras, les substances médicales ou pharmaceutiques, plus particulièrement les substances antimicrobiennes, de préférence les antibiotiques, les désinfectants, les substances anti-inflammatoires, les substances favorisant la cicatrisation, les substances analgésiques, les substances de lutte contre les odeurs, les facteurs de croissance cellulaire, les facteurs de différenciation cellulaire, les facteurs de recrutement cellulaire et/ou les facteurs d'adhésion cellulaire, les plastifiants, et leurs combinaisons.

10. Procédé selon l'une quelconque des revendications précédentes, **caractérisé en ce que** les corps façonnés sont interconnectés au niveau facial par séchage, plus particulièrement à une température située dans la plage comprise entre 20°C et 80°C, plus particulièrement entre 25°C et 45°C, et de préférence entre 30°C et 40°C.

11. Procédé selon l'une quelconque des revendications précédentes, **caractérisé en ce qu'**un implant de type corps creux, de préférence un implant tubulaire, est produit par interconnexion au niveau facial des corps façonnés sur le support, de préférence sur un support de forme cylindrique ou conique.

12. Procédé selon l'une quelconque des revendications précédentes, **caractérisé en ce que** le produit est réticulé, par exemple dans une étape de traitement subséquente et de préférence en présence d'un plastifiant.
